# EUROPEAN PATENT SPECIFICATION

(11) **EP 4 161 388 B1**
(45) Date of publication and mention of the grant of the patent: **02.09.2026**
(21) Application number: 21729895.9
(22) Date of filing: 02.06.2021
(51) Int. Cl.: A61B 6/00, A61B 6/58, G01N 23/041, G21K 1/04, G21K 1/06

(54) **STEPPING STRATEGY FOR DEFECT COMPENSATION IN DAX IMAGING**
SCHRITTSTRATEGIE ZUR FEHLERKOMPENSATION IN DAX-BILDGEBUNG
STRATÉGIE D'ÉTAPE POUR LA COMPENSATION DES DÉFAUTS DANS L'IMAGERIE DAX

(30) Priority: 08.06.2020 EP 20178712
(43) Date of publication of application: 12.04.2023
(73) Proprietor: Koninklijke Philips N.V., 5656 AE Eindhoven (NL)
(72) Inventor: PRALOW, Thomas, 5656 AE Eindhoven (NL); KOEHLER, Thomas, 5656 AE Eindhoven (NL); LOESCHER, Stefan, Leonhard, 5656 AE Eindhoven (NL)
(74) Representative: Philips Intellectual Property & Standards
(86) International application number: PCT/EP2021/064725
(87) International publication number: WO 2021/249836

(56) References cited:
- EP-A2- 3 391 821
- WO-A1-2014/027289
- DE-A1- 102016 211 761
- US-A1- 2015 092 914

## Description

### FIELD OF THE INVENTION

The invention relates to an imaging apparatus for dark-field X-ray imaging and/or phase-contrast-imaging.

### BACKGROUND OF THE INVENTION

Dark-field imaging has attracted much interest especially in the medical field. Dark-field X-Ray ("DAX")-imaging is a type of X-ray imaging. Contrast in dark-field imaging relates to the amount of small angle scatter experienced by the X-radiation.

Experimental dark-field imaging with mice have been reported by A. Yaroshenko et al in "Pulmonary Emphysema Diagnosis with a Preclinical Small-Animal X-ray Dark-Field Scatter-Contrast Scanner", Radiology, vol. 269, No 2, November 2013. Another example of an apparatus for dark-field imaging is shown in US2015/092914. This apparatus shows a device with a periodic structure with a spatial period in a first direction, and a phase stepping mechanism displacing this device in the first direction.

DAX imaging may be used to detect lung diseases like COPD (Chronic obstructive pulmonary disease), fibrosis etc. In particular for early detection of COPD, quantitative measurements may be desirable.

DAX imaging, but also phase contrast imaging, may rely in implementations on an interferometer including one or more gratings. One or more of the gratings is moved relative in a phase stepping operation relative to an X-ray beam of used in the imaging.
Phase contrast and/or DAX imagery may on occasion include image artifacts which is undesirable.

### SUMMARY OF THE INVENTION

There may therefore be a need for improving phase contrast and/or DAX imaging.

The object of the present invention is solved by the subject matter of the independent claims where further embodiments are incorporated in the dependent claims.

According to a first aspect of the invention there is provided an imaging apparatus including at least one device for phase contrast and/or dark field imaging having a periodic structure with a spatial period, and further including a phase stepping mechanism configured to facilitate a relative phase stepping motion between the at least one device and a focal spot of an X-ray source of the imaging apparatus, the relative phase stepping motion to cover an (effective) distance greater than the said spatial period.

The proposed imaging apparatus helps reducing or eliminating image artifacts in phase contrast and/or DAX imagery. The image artifacts may stem from local imperfections (defects) in the imaging facilitator device. The artifacts may occlude one or more detector pixels and may disturb the spatial periodicity of the imaging facilitator device.

The imaging facilitator device has in general (spatially) periodic structures with period *p.* For example, in grating based embodiments, the period refers to a set of parallel trenches formed in a suitable substrate of a grating. In coded apertures, the period may refer to the spatial periodicity of the apertures, and so on for imaging facilitator devices of other types. The said defects may have been caused during the manufacturing of the imaging facilitator device. The defects may occlude certain detector pixels or cause detection at reduced contrast during phase stepping, which in turn may then lead to image artifacts when computing by an image generation algorithm the phase contrast and/or DAX imagery.

The phase stepping motion may be imparted in a single sequence of multiple steps, or in multiple such sequences with different starting point(s).

In embodiments, the said distance is greater than a multiple of the said period but is not a multiple of the said period.

In embodiments the said distance is at least twice a pixel size of a detector of the imaging apparatus.

In embodiments, said distance is based on a size of a defective region in the periodic structure.

In embodiments, the phase stepping mechanism is configured to impart the phase stepping motion in one or more steps having at least one width, wherein said at least one width is greater than, the said period.

In embodiments, the said step width is based on a size of a defective region in the periodic structure.

In embodiments, the device is assembled from sub-modules giving rise to one or more gaps in the device, and wherein the said defective region includes the said one or more gaps.

In embodiments, the phase stepping mechanism includes a frame in which the device is suspended, and an actuator configured to cause the said phase stepping motion.

In embodiments, there is a single such actuator.

The spatial period is along a first direction, wherein the phase stepping motion has a displacement component along a second direction at an angle to the first direction and a displacement component along to the said first direction. The displacement along the first direction has a step width greater than, or greater than a multiple of, the said period. This allows collecting sufficient number of measurements not occluded by a defect in the device for phase contrast and/or dark field imaging. The displacement allows avoiding occlusion by the defect if this or other defects has/have an appreciable spatial extent in the second direction. The second direction may be perpendicular to the first direction.

In embodiments, the phase stepping motion is along a curve.

In embodiments, the device is suspended in the frame at at least two suspension points by a respective flexure bearing.

In embodiments, the at least one of the flexure bearings has a flexure element angled at 40-50° relative to the said first direction.

In embodiments, the device includes an interferometric grating.

*"Phase retrieval (algorithm)"* as used herein is a type of image generation algorithm for generating phase contrast or dark-field imagery. The algorithm may be based on signal models or otherwise that allow computing the phase contrast image and/or the dark-field image from measured intensities. Because of the mutual interplay between attenuation, phase shift and the dark-field signal, which results from small angle scattering, in phase retrieval algorithms all images, attenuation, dark-field and phase contrast, are usually computed jointly, although this may not necessarily be so all implementations. Although "phase retrieval" is an established name, it may also be referred to herein as a "dark-field (signal) retrieval". The phase retrieval operation may be facilitated by a dark-field or phase contrast imaging facilitator device such a grating(s), a structured mask, a coded aperture plate, a crystal etc, or other at least partially radiation blocking structures with periodic or non-periodic sub-structures, that interact with the imaging X-ray beam to realize different intensity measurements at the detector to so impose more constraints. This helps resolving ambiguities, or ill-posedness, otherwise inherent in phase retrieval. The image generation algorithm may include fitting the signal model to the measured data (also referred to herein as projection images/data). The fitting procedure may be formulated as an optimization problem. The optimization is to improve a cost function that measures a mis-fit between values as per the signal model and the measured data.

*"Phase stepping"* as used herein is a process to realize the said different measurements using the imaging facilitator device. Phase stepping may include moving the imaging facilitator device relative to a focal spot of the X-ray source used for imaging.

By *"pixel position"* is either meant a native pixel position on a detector for a detector pixel, or a location for a geometrical ray (extending from the X-ray source) that defines a location in image domain where the dark-field image is to be generated or "reconstructed".

The imaged *"object"* is animate and includes a human or animal or a part thereof, or the object is inanimate such as an item of luggage in a security screening system or a sample object in non-destructive material testing, etc.

### BRIEF DESCRIPTION OF THE DRAWINGS

Exemplary embodiments of the invention will now be described with reference to the following drawings, which, unless stated otherwise, are not to scale, wherein:
Fig. 1 shows schematically an X-ray imaging arrangement configured for phase contrast and/or dark field imaging;
Fig. 2 shows an example visibility map with projection footprints of grating defects;
Fig. 3 shows schematically a phase stepping operation;
Fig. 4 shows schematically in projection view a grating including gaps;
Fig. 5A-C show schematically different embodiments of a phase stepping mechanism;
Fig. 6 shows schematically a suspension system as used in a phase stepping mechanism according to one embodiment;
Fig. 7 shows schematically a flexure bearing as may be used in a suspension system of a phase stepping mechanism according to one embodiment; and
Fig. 8 shows a flow chart of a method of phase contrast and/or dark field imaging.

### DETAILED DESCRIPTION OF EMBODIMENTS

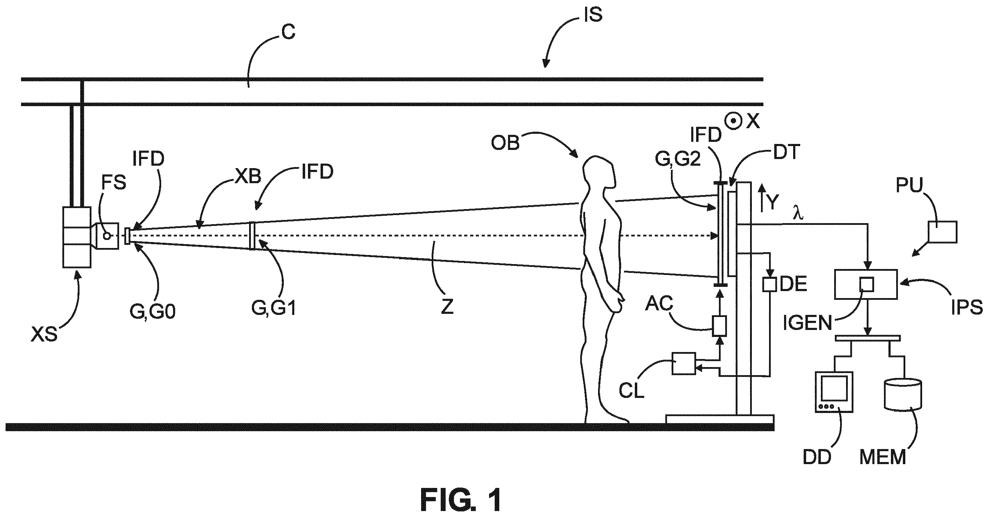

With reference to Fig. 1, there is shown a schematic block diagram of an imaging arrangement IR that includes a computerized image processing system IPS and an X-ray imaging apparatus IA ("imager"). The X-ray imaging apparatus is configured for dark-field X-ray ("DAX"-) imaging and/or phase-contrast ("Φ"-) imaging. The image processing system IPS may be implemented by on one or more processing units PU such as one or more computers, servers, etc.

Broadly, the image processing system IPS includes an image generator IGEN that processes projection imagery λ acquired by the imager IA into dark-field and/or phase-contrast imagery. As will be explored more fully below, the imager IA is configured herein to reduce or to entirely eliminate certain artifacts in phase contrast and/or dark field imagery. Imagery as provided by the image generator IGEN can then be displayed on a display device DD or can be stored in a memory MEM for later review or use, or it can be otherwise further processed.

Although in Fig. 1 it is envisaged that the imaging apparatus IA supplies the projection imagery λ directly, via wireless or a wired connection, to the image processing system IPS, this may not be so in all embodiments. For instance, the projection imagery λ may be first stored in a memory such as in a picture archiving system (PACS) of a hospital information system (HIS) or otherwise, and the imagery to be processed is retrieved at a later stage (e.g. upon user request) and is then processed by the image processing system IPS.

In more detail, the imaging apparatus IA includes an X-ray source XS and an X-radiation sensitive detector DT. The X-ray source XS is arranged opposite the detector DT with an examination region defined therein between. The apparatus may further include an DAX/Φ-imaging facilitator device IFD to facilitate phase contrast and/or DAX-imaging, such as an interferometer, arranged in the examination region between the source XS and the detector DT. For brevity, the DAX/Φ-imaging facilitator device IFD may be referred to herein simply as the "imaging facilitator device IFD". The apparatus IA may be arranged for chest imaging as shown in Fig. 1 where the patient OB is standing in the examination region between source XS and detector DT during imaging. The source may be ceiling C mounted (as shown) or may floor mounted in a stand or may be mounted in any other way conducive to the imaging task at hand. The detector DT may equally be ceiling C mounted, or floor mounted on stand as shown in Fig. 1.

The imaging axis Z is oriented from the source XS to the detector DT as shown in Fig. 1. The source and the detector may be arranged in a common gantry such as when the imager IA is of the C-ram or U-arm type, or is a CT scanner. The imaging axis Z is an imaginary line that runs from the focal spot FS of the source XS to a center point of the x-ray sensitive surface of the detector DT. The surface is formed by a plurality of X-ray sensitive detector pixels.

The imaging apparatus IA may be of the full field-of-view (FoV) type as shown in Fig. 1, where the detector is of the flat panel type. In full FoV imaging system, the size of the detector DT and at least a part of the size of the IFD corresponds to the desired FoV. Alternatively, the detector DT and the imaging facilitator device IFD may be smaller than the intended FoV such as in slot-scanning systems.

During imaging, the X-ray source XS is activated to generate an X-ray beam XB which is detectable by the detector DT. The x-ray beam XB propagates along imaging axis Z, interacts with patient tissue and is then detected at pixels of the detector DT. The X-ray beam XB is caused by an electron beam generated inside the source XS. The electron beam is accelerated towards, and impacts on, an anode at a focal spot FS from which the X-radiation then issues forth.

The imaging facilitator device IFD, or a group of such devices, allows translating X-ray beam XB refraction and/or small angle scattering of the beam XB into intensity modulations at the detector DT, thereby facilitating resolving said modulations into dark-field and/or phase-contrast image signals and, if desired, into an attenuation image signal. The imaging facilitator device IFD may include physical imperfections that may cause image artifacts in the phase contrast or dark-field image. The imaging apparatus IA and/or the image processing system IPS proposed herein is configured to reduce or eliminate such image artifacts.

In the following, main reference will be made to an interferometric imaging apparatus IA including the interferometer as the imaging facilitator device IFD, although this is not to exclude embodiments that use other, in particular non-interferometric, imaging facilitator devices IFD. Such non-interferometric imaging facilitator devices IFD include for example coded aperture systems. In general, the dark-field or phase-contrast is obtainable by the imaging facilitator device IFD causing a periodic wave front modulation on the incoming imaging X-ray beam and by measurements, by the X-ray detector DT, of a variation of the resulting wave-front caused the object OB to be imaged.

Turning now in more detail to the imaging apparatus IA, this can be a configured for 2D imaging such as a radiography apparatus or for 3D imaging such as a CT scanner. The object OB (e.g., the chest of the subject) to be imaged resides in the examination region during imaging. In the examination region there is arranged the interferometer as one embodiment of the imaging facilitator device IFD. The interferometer includes a single, two or three (or more) grating structures. In the embodiment, the interferometer includes three gratings. As said above, the interferometer is but one embodiment of the imaging facilitator device IFD and we will make main reference to this embodiment in the following, with the understanding that the principles of the present disclosure are not confined to interferometry but can be readily extended to other grating-based or non-grating based structures as other embodiments of the imaging facilitator device IFD.

With continued reference to the (non-limiting) interferometric embodiment of the DAX/Φ-imaging facilitator device IFD, periodicity, aspect ratio, etc. of the gratings are such that they cause diffraction of the X-ray beam and/or just enough coherence is achieved so that the small-angle scattering can be detected or derived. Absorption and phase gratings may be used. In one embodiment the gratings are formed by photolithography or cutting in silicon wafers to define a periodic pattern of parallel trenches. In the absorption gratings, the trenches may be partly or fully filled with lead or gold, or other material that is non-transparent for x-radiation, to form as set of lamellae. The lamellae or the trenches may be referred to herein as the (grating) structures ST. The structures ST extend in parallel in a longitudinal first direction X. There is a direction Y across the structures ST and across the first direction X. There is a spatial frequency of the structures with a periodicity p along direction Y. The directions X, Y as defined above are in general perpendicular to the imaging axis Z. As will be explored below in more detail, in some embodiments, in order to enable DAX and/or phase contrast imaging, the imaging facilitator IFD, such as one or more of the gratings, is moved during X-ray exposure along a scan path. This motion may be referred to herein as the phase stepping (motion). The scan path describes a motion relative to the focal spot FS. The scan path is either along the said direction Y or has, at least at times, a directional component along the Y direction.

In alternative embodiments to a mechanical motion of the imaging facilitator device IFD, the scan path is realized instead by electric means to move the focal spot of the source XS. Moving the focal spot may be achieved by diverting, through an electrostatic arrangement, the electron beam so it that it impacts the anode surface at a different location. Alternatively, the anode is mechanically moved, or the whole X-ray source XS is moved mechanically. Alternatively still, a collimator (not shown) is used to define the scan path.

The gratings may be planar or may be curved for better signal efficacy to form portions of lateral surfaces of imaginary concentric cylinders with their common axis passing through the focal spot FS of source XS. In Fig. 1, the said axis extends into the plane of the drawing. The inter-grating distances, the period and aspect ratio are in general a function of the design energy. In Talbot-Lau interferometer, the distance between gratings G1 and G2 is a Talbot distance of a desired order, e. g. of first order.

In yet more detail and in one embodiment, an absorbing grating structure G2 is arranged between the detector DT and the object OB whilst the other grating G1, a phase grating, is arranged between the object OB and the X-ray detector DT. In some embodiments there is also an additional grating G0 arranged at the X-ray source XS, in case the X-ray source is incapable of generating natively sufficiently coherent radiation. Otherwise if radiation is sufficiently coherent, grating G0 may be omitted. If the X-ray source produces incoherent radiation (which is usually the case), the (absorption) grating G0 at the X-ray source (also referred to as the source grating) transforms the X-radiation coming out of the X-ray source into an, at least partly, coherent radiation beam XB. Inverse geometries where G1 is placed upstream the object OB, i.e. between source XS and imaged object OB, are also envisaged. Each of the gratings G0, G1, G2 has in general a different period *p*₀, *p*₁, *p*₂. In the following, a reference herein to a "grating G" with "period *p"* should be constructed as a generic reference to any of the three gratings G0-G2, with period *p* an generic reference to their respective periodicities *p₀, p₁* or *p₂.*

The at least partly coherent radiation beam XB propagates through the examination region along imaging axis Z and interacts with the interferometer IFD and the patient OB. After said interaction, the radiation is then detected in form of electrical signals at X-radiation sensitive pixel elements PX of the detector DT. Data acquisition circuitry (not shown) digitalizes the electrical signals into projection (raw) image data λ which is then processed by the IPS in a manner explained in more detail below.

The image generator IGEN outputs the dark-field signals and/or the phase-contrast signals as respective arrays of image values which form the dark-field image and the phase-contrast image, respectively. These image values or pixel values represent respectively the contrast for the dark-field signal and the phase-change experienced by the X-radiation while traveling through the object OB along the respective geometrical ray.

Generally, when X-radiation interacts with material, it experiences both, attenuation and refraction and hence phase change. The attenuation on the other hand can be broken down into attenuation that stems from photo-electric absorption and attenuation that stems from scatter. The scatter contribution in turn can be decomposed into Compton scattering and Rayleigh scattering. For present purposes of dark-field imaging it is the small angle scattering that is of interest, where "small angle" means that the scatter angle is so small that the scattered photon still reaches the same detector pixel as it would have reached had it not been scattered at all.

The dark-field contribution can be modelled in terms of visibility *V* = *Vₒ e* ^{*-*∫ *ε*(*z*)*dz*}, with *ε* is the spatial distribution of the diffusion property of the patient OB and the integration is performed along the respective x-ray beam path through the object, and *V*₀ being the reference visibility without object interaction (recorded in a calibration measurement). The dark-field signal as recorded in the dark-field image is then *D* = *V*/*V*₀*.* The (differential) phase contrast can also be modeled as a line integral as has been reported elsewhere.

Traditional X-ray systems are usually incapable of resolving the detected signal into dark-field or phase contrast contribution. However, by using the interferometer G0-G2 as shown in Fig. 1, or by using other imaging facilitator devices IFDs, it is possible to translate these contributions into an intensity pattern of fringes which can be analyzed by the image generator IGEN to obtain the phase-contrast and/or DAX image.

Turning now in more detail to the image generator IGEN, this operates on a series of projection images obtained in the above mentioned phase stepping operation. The projection imagery λ thus includes intensity measurements *Mᵢⱼ* of each pixel *i* and phase step *j*. In other words, each pixel *i* records a plurality of different measurements, *Mᵢⱼ.* Based on this recorded series of projection images, the image generator IGEN computationally resolves the detected fringe pattern in the series of projection data into three contributions or signal components, namely the refraction contribution (also referred to as the phase-contrast signal), the dark-field signal component and the remaining attenuation component.

Because of these three contrast mechanisms acting together, the signal processing by the IGEN of the detected series of intensities proceeds in three signal channels (phase-contrast, dark-field, and attenuation). In the above described types of imaging system, the capability for dark-field/phase-contrast imaging is achieved as follows: the projection data is acquired at the detector DT during the phase stepping operation as a series for a given fixed projection direction. Conventionally, the phase of the fringes is typically stepped over 360°, a full phase of the period *p.* As will be explained below in more detail, the phase stepping is preferably extended beyond a single phase in departure of such conventional phase stepping strategies. The phase stepping operation is realized by a phase stepping mechanism PSM that induces a motion between focal spot FS and the imaging facilitator device IFD, or a component thereof. For instance, in one embodiment the analyzer grating G2 (that is, the grating arranged between object and detector) or another one of the gratings G0,G1 is moved ("scanned") laterally along the scan path relative to the focal spot FS. Alternatively, as mentioned above, the phase stepping can also be achieved by moving the focal spot FS of the X-ray source.

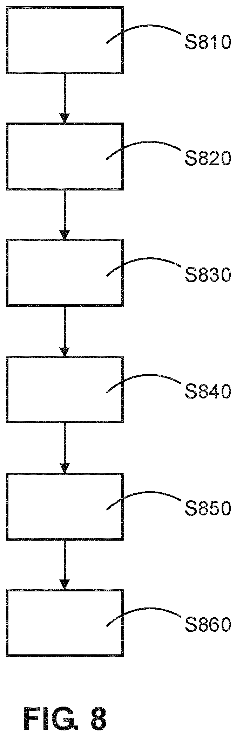

The phase stepping motion *j* causes a change of the fringe pattern which in turn can be recorded at the detector DT in the corresponding series for each step of the motion. This series of measurements *Mᵢⱼ* form, for each geometrical ray/pixel *i,* an associated phase curve. The phase curves are in general of sinusoidal shape and has been found to encode the quantities of interest, in particular the dark-field signal, along with attenuation and phase change. Details of the image generation algorithm will be explored more fully below at Fig. 8.

Turning now to the phase stepping mechanism PSM in more detail, this includes an actuator AC such as a servo or stepper motor, and a control logic CL to control operation of the actuator AC. The actuator AC engages with the grating G to be moved and causes same to proceed long the scan path. The scan path may be lineal or may be curved as will be explained in more detail below.

The actuator AC causes in embodiments a stepped motion of the grating G at a step width. The steps are synchronized with switching the X-ray source XS to cause synchronized exposures that form the phase stepping measurements. Alternatively, the x-ray source XS is not so switched but operates with continuous exposure during the phase stepping and the different measurements so obtained are indexed with the respective phase steps of the grating G. However, a stepped motion performed by the actuator AC is not necessarily required in all embodiments to implement the phase steps, as the actuator may be arranged instead as a server motor that causes a constant motion of the grating G along the scan path with switching of the x-ray source at an adjustable frame rate to obtain the phase stepping measurements. In embodiments with constant motion, the X-ray exposures are synchronized to that the distance travelled by the grating between exposures is as explained above greater than the grating period of the grating having the defect. The motion and synchronization results in an effective phase stepping with the correct effective step width.

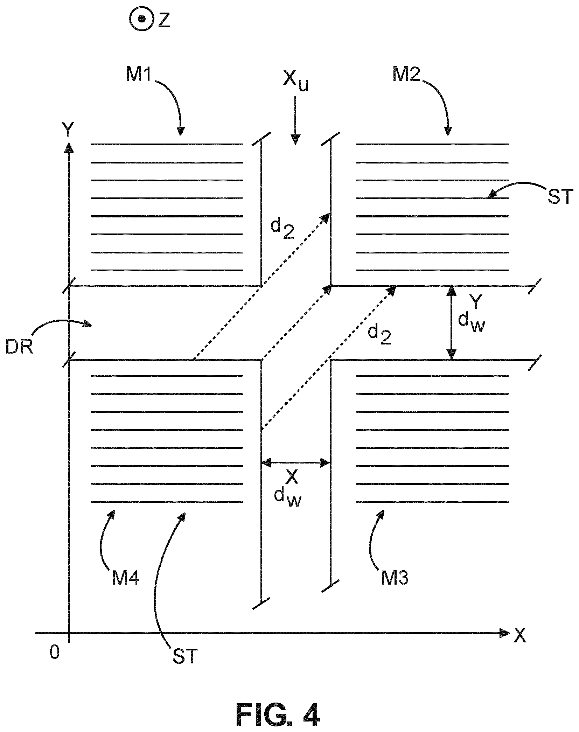

Applicant has observed that image artifacts may be incurred due to imperfections in the gratings themselves. The imperfections, referred to herein as "defects" or "defective regions" DR, may be random or systemic. An example of a random defective region of a grating G may be caused unintended in the manufacturing of the grating. For example, residual material, such as particles of the fill material that are used to from the lamellae, may remain outside the trenches to disturb the periodic pattern. Other such residual material may include particulates, "seeds", that are left by the lithographic process of the trenches. Alternatively, or instead, foreign particular matter may lodge into the grating structures to cause a defective region to be formed. The size of the defective regions may be in the micron range. It may cover several periods *p* of the grating. Alternatively, the defective region DR may cover substantial parts of the grating surface such as may be the case for defective regions of the systemic type. An example for such a systemic defective region DR includes gaps that are necessitated by the way in which the grating is manufactured. For example, the G2 grating, which is the largest of the gratings used in the interferometric set-up, is required in full-view chest imaging to substantially conform with the size of a human lung. Accordingly, analyzer gratings G2 could cover a surface area in the order of 50x50cm². As of today, it is difficult or impossible to produce a G2 grating of such size in one piece. In certain existing manufacturing techniques therefore, the whole grating G2 is intentionally assembled from sub-gratings or sub-modules M1-4 (shown in Fig. 4) that are joined together to leave a system of gaps that extend in X and Y direction to form a grid-shaped defective region. The width of the joining gaps may be in the order of 50 microns and they too may extend to over several grating periods.

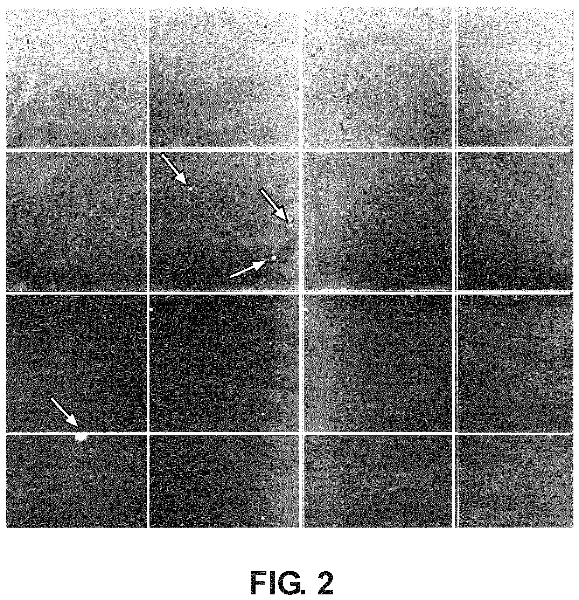

Defective regions of gratings are illustrated in the visibility map as shown in Fig. 2. The visibility map shows the visibility *V*₀ over the detector DT and is derived from a series of projections recorded in a calibration procedure by the detector DT without there being an object present in the examination region. The visibility map records projection footprints of the various defective regions in the gratings as bright spots (that represent low intensity) of different sizes and shapes. The spots referenced by the small arrows represent random defective regions. The grid shaped pattern is the projection footprint of the above mentioned gaps that are due to assembling the grating G2 from smaller gratings, the said sub-modules M1-4. In the example there are sixteen such sub-modules arranged in a 4 x 4 layout. It will be understood that each of the gratings in the interferometer G0-G2 may be marred by such defective regions. For instance, in the visibility map in Fig. 2, the bright spot at the lower left indicated by the larger arrow stems from the source grating G0 whilst the grid artifact in form of the smaller spots indicated by the three other arrows stem from defects in the G2 grating. If unaccounted for, the defective regions DR cause spurious values measurements to be recorded by the detector during phase stepping which then may lead to incorrect phase contrast and/or dark field imagery being generated with artifacts. It is therefore proposed herein to have the control logic CL cause the actuator AC to move the respective grating G along a scan path according to a suitable phase stepping strategy to mitigate, if not entirely eliminate, such artifacts. Suitable such scan path strategies are explored in more detail below. More than one of the gratings may be moved in the phase stepping. Preferably, the source grating G0, the smallest in size, is moved by the actuator AC. Alternatively or in addition, it is the largest of the gratings, the analyzer grating G2, that is moved by the actuator AC. The phase grating G1 may be moved instead or in addition. Any combination of motions, effected by a set of actuators, is envisaged herein. Preferably however, only a single one of the gratings is moved such as G0 or G2 relative to other(s). In embodiments where the focal spot FS is moved instead, the control logic CL controls a suitable actuator (not shown) at the X-ray source XS, or controls the electric means to deflect the electron beam in realizing the scan path.

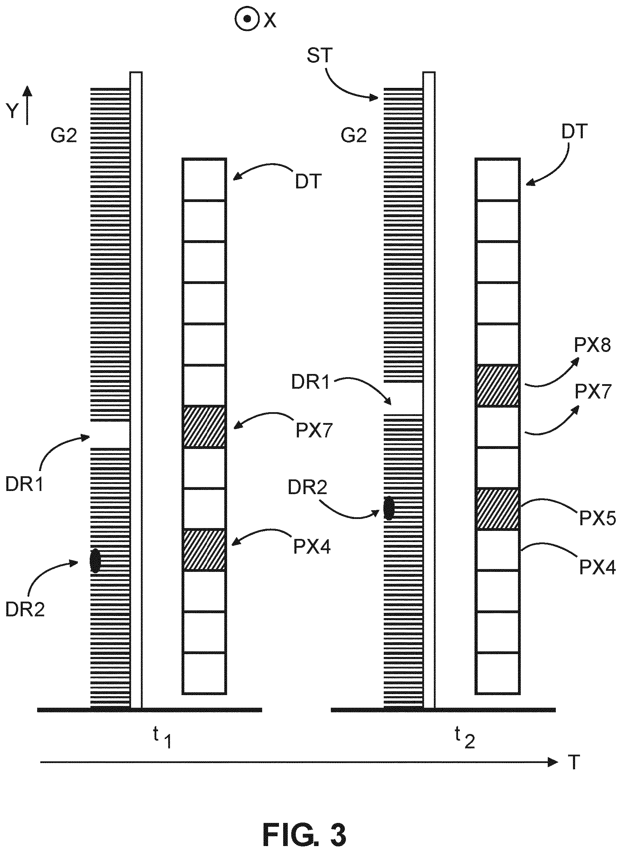

Fig. 3 is a further illustration as to how defective regions DR1, DR2 in a grating G, such as the analyzer grating G2, can affect the detector readings at different pixels PX*ᵢ*. Fig. 3 shows views along the grating structures ST in X direction at different times t1, t2 during the phase stepping performed along a lineal scanning path in Y direction.

In the Figure there is schematically indicated a systemic defective region DR1, such as one of the gaps between adjacent modules M1, M2, and a random defective region DR2 such as a residual particulate that remained after etching the grating trenches or filling same for example. At time t1, it is pixels PX7 and PX4 that record spurious measurements due to the respective defective regions DR1, DR2 occluding the respective pixels PX7, PX4. The occlusions are because the defective regions DR1, DR2 happen to be residing in projection view along Z above the respective pixels PX7, PX4. However, with one of the new phase stepping strategies as envisaged herein, if the phase-stepping width between consecutive measurements is large enough it may be assured that previously occluded pixels PX4, PX7 are eventually no longer occluded thus capable of recording correct measurements, which, of course, is now at the expense of neighboring pixels PX7, PX5 and PX8 which are now occluded instead. By choosing a phase stepping strategy with a suitable phase step width so as to spread the phase stepping measurements over a larger spatial region to cover a distance of more than a grating period, it can be ensured that each or most pixel will actually "see", that is record, a sufficient number of measurements. Having sufficient number of such measurements in turn allow a robust calculation of the phase contrast and/or dark field contribution. The number of phase steps is preferably more than three. The step width may be more than one period p of the moved grating. For example, the step width may be more than a multiple (for example, more than twice) the period p but preferably should not equal a multiple of the period. It can be generally said that to ensure a robust calculation of the phase contrast and/or dark field image, each pixel position PX*ᵢ* should on average see at least three different non-occluded measurements. The width of the individual steps during the phased stepping, or more generally, the total distance covered by the grating G during the phase stepping, will thus depend on the size of the grating defects to be expected in all gratings G0-G2 used. As said, the phase stepping distance covered in a given step can be expected to be in the order of multiple grating periods of the grating G to be moved. This is because in general the spatial dimensions of a defective regions DR, that is, its size in X or Y direction, is larger than the periodicity *p* of the grating G. The order of magnitude of defective regions ranges from the same order of magnitude as the periodicity *p* to one or two orders of magnitude higher. Defects with dimensions smaller than the grating period *p* are of lesser concern herein.

Fig. 4 shows schematically a projection view along the imaging axis Z of a portion of a defective region DR in the form of the above described grid of gaps that are caused by manufacturing the grating G, such as G2, from sub-modules M1-M4.

Where corners of modules M1-4 meet, the gaps form cross-shaped patterns as shown in the projection view of Fig. 4. Gaps widths ${d}_{w}^{Y} , {d}_{w}^{X}$ along the two directions X, Y, respectively are generally larger than the periodicity *p* of the grating G. Preferably therefore, to promote acquisition of a sufficient numbers of non-occluded measurement per pixel PX*ᵢ*, the distance covered during phase stepping operation in a step along direction Y, assuming for now a lineal scan path, should at least be ${d}_{w}^{Y}$, the width of the gaps along direction Y. However, if phase stepping strategies are confined to (lineal) scan paths along direction Y, across the grating structures ST, there will then remain a large number of pixels, which will never record a non-occluded measurement because they are occluded at all times by an intervening gap. Such permanently occluded pixels as indicated in the Figure with coordinates Xᵤ.

It is therefore proposed herein to use a scan path that does not only have a directional component in Y direction across the grating structures ST, but also in addition a component parallel to the longitudinal orientation of the structures ST in X direction. A curved scan path may be used such as along a circular arc to ensure that there is a sufficient motion component along the grating structures ST in X direction to avoid permanently occluded detector pixels, in addition to a component for phase stepping along Y across the structures' ST periodicity. As indicated in Fig. 4, a diagonal scan path at 45° along a diagonal in the XY co-ordinate system may be used to ensure that sufficient non-occluded measurements can be collected. Preferably, the distance *d2* covered is proportional to $d \sqrt{2}$, with *d* the average width of the gaps. Preferably, the distance *d2* equals or is at least $2 d \sqrt{2}$ to account for the relatively long stretches of occlusion tangential to the corner portion of the grid modules.

It will be seen from the above Figs. 4,3 that the preferred phase stepping strategies as envisaged herein implement scan paths that cover in total and per step a distance of more than a multiple of the grid period *p,* so as to ensure that a sufficient number of non-occluded sample measurements are collected for each pixel or at least to increase the number of such pixels that are not-occluded at all times during phase stepping. Whilst it may not be possible to ensure that all pixels measure the required number of measurements, with the proposed strategy artifacts that stem from occluded pixels can at least be reduced. As each of the gratings G0-G2 may include defects that may unfavorably contribute to the above described occlusions, each of the gratings G0-G2 may be subjected to respective phase steps, with respective step width larger than the respective period *p₀,p₁,p₂.* If a non-lineal, such as curved path is used, it is preferable that at some or each step, the displacement component along direction Y is greater than the period or specifically greater than a multiple of the period p.

As mentioned above, a diagonal scan path with displacement components along X and Y may be desirable as this allows uniformly sampling across and parallel to grating periods. The length of the step width for scan path is in general a function of the maximum or at least average size (along X and/or Y) of the defective regions DR.

For systemic defective regions, such as the grid of gaps in Fig. 4, the required phase stepping distance and/or the phase step width may be a function of at least one, preferably both, of ${d}_{w}^{Y} , {d}_{w}^{X}$*.* The gap widths ${d}_{w}^{Y} , {d}_{w}^{X}$ may be readily established by consulting grating manufacturer's tolerances or specifications. So at least for systemic defects, such as the said gaps, the tolerances may be known with sufficient accuracy a priori. A controller CL to control the actuator AC may then be programmed to set the correct step width/total phase stepping distance. This can be done in a one-off setup operation, or whenever the gratings are replaced.

However, for random defects DR, the imaging system may include a logic such as a defect evaluator DE that helps establish the requisite phase step width for any given grating set up. Specifically, in an initial calibration phase, a visibility map as mentioned above in relation to Fig. 2, is acquired by detector DT. The defect evaluator DE uses image processing, such as a segmentation algorithm, to identify footprints of defects. Intensity thresholding may be used to identify singular bright spots, such as illustrated above in Fig. 2. The detector evaluator DE uses a suitable metric to quantify the size of the projection footprints of the grating defects as found in the segmentation. The defect evaluator DE calculates an average size, or a maximum size, of the defects so found. The sizes are preferably for each of the two spatial dimensions X and Y. The metric may be stored in a memory. The metric may be used to control the controller CL of the phase stepping mechanism so as to instruct the actuator AC to implement the correct phase stepping width. For instance, in embodiments the phase stepping width or total distance covered is chosen at least or larger than the maximum size of defects so found. Alternatively, the phase stepping width or total distance covered is set to an average size of the defects so found. The displacement in X direction is also adjusted to set the curved or diagonal scan path. If occlusion along X direction can be neglected, the detector evaluator DE may use a negligibly threshold to establish this, only the size of the defects in Y direction are recorded and a conventional lineal scan path solely along Y direction may be used to cover the requisite grating periods per step based on the computed metric along direction Y.

The defect evaluator DE may be implemented as part of the general image processing system IPS. As said, the calculation of the requisite step width may be done as a one/off operation at the set-up of the imaging system IS or may be repeated whenever a grating G0, G1, G2 is be to replaced.

In the following, various phase stepping strategies are now explored in more detail. In general, the phase stepping strategies envisaged herein are configured to "spread" the measurements in respect of the stepped grating G over a larger than usually necessary spatial area so that each (or at least more) pixel(s) can collect a requisite number of measurements without being occluded by a defect. Previously, the phase stepping is done only to cover a distance of a whole grating period *p.* However, in the proposed phase stepping strategy, a distance greater than one grating period *p* is covered. Two different concepts are envisioned herein, wherein G denotes the grating to be stepped and *p* its spatial period of the grating pattern. The phase stepping strategies envisaged broadly include block-stepping strategies and distributed stepping strategies.

In block-stepping strategies the grid G is stepped in a conventional manner along direction Y to cover one period of the grating, starting at an arbitrary location *x*₀, *y*₀ in the X,Y co-ordinate system. Then, subsequently, in another block, another conventional phase stepping operation is performed with a certain number of steps, e. g. four, to again cover the period p, but this time with grating G at a different start position *x*₀'= *x*₀ + *Δx, y*₀' = *y*₀ *+Δy.* The measurement results from these two phase stepping operations are then merged to obtain enough non-occluded measurements per pixel. It may not be necessary however that both phase stepping operations cover the whole period, but this is preferred as described. More than two blocks of such phase stepping operations may be done.

This block-stepping approach may attract additional dose unless the measurements are processed in a fitting operation (discussed in more detail below at Fig. 8) in order to allow such merging from the two phase stepping blocks. However, such processing may not be required if the starting point for the second or subsequent block is offset by *M p* in Y-direction, *M* being an integer *M* = 1,2,3... Now, the measurements from two blocks can be merged and no extra dose is required.

In distributed stepping strategies, there is a single stepping sequence without interruption and moving to a new starting point *x*₀', *y*₀'. In distributed stepping, the phase steps by which the grating G is displaced are spread over multiple grid-lines, adding an *M p* term in the Y direction to some or each of the individual phase steps. In other words, the phase stepping width is enlarged by *M p* for some or each step in a single sequence of steps for a given phase stepping operation.

The scan path may be lineal along direction Y perpendicular to the course of the grating structures ST along X. Alternatively, as will be explored in more detail below at Fig. 4, the scan path is curved so is not always along direction Y but has displacement components parallel to grating structures (grating lines) along direction X. This allows collecting non-occluded measurement in case of defects with appreciable spatial extent along direction X. In embodiments, the scan path does not necessarily have to be curved to have displacement along direction X. For example, the scan path may still be lineal but is at an angle to the grating lines, such as a lineal diagonal scan path in the X, Y-coordinate system.

A main idea for the distributed phase stepping embodiment may now be formulated in more detail. In conventional phase stepping, measurements are taken with grating G at different positions along Y, with *j* non-negative integer ranging from 0 to *N-1:* ${y}_{j} = {y}^{\left(0\right)} + \frac{jp}{N}$
where *y* is the direction perpendicular to the grating lines. However, due to periodicity of the grating structures ST, it is also possible to displace the grating G as per: ${y}_{j} = {y}^{\left(0\right)} + \frac{jp}{N} + Mjp$
for integer *M* to generate the same phase stepping. In general, the step width is greater than the period p, but is not a multiple of the said period p. For example, as in (2), a step width of
p (1/N+M) may be chosen to fulfill these conditions. However, herein step width p (1/N+M) is merely an exemplary formulation and not to limit the present disclosure.

The integer *M* causes the enlarged step width. If *M* is chosen large enough, it can be ensured that a defect footprint is projected onto different detector pixels and hence its adverse occlusive effect on image quality can be reduced. On the other hand, it is preferable to keep *M* as small as possible since an unnecessary large step width *M* will require more time for the grating movement and may require additional grating area. Therefore, it is desired to select the enlarged phase step (defined by the term $\frac{jp}{N} + Mjp$ in (2)) such that a phase retrieval is robust enough, even if a defect occludes the same pixel for a number of adjacent physical stepping positions. For instance, if eight phase steps are taken, a sequence of ${y}_{j} = {y}^{\left(0\right)} + \frac{{F}_{j} p}{N} + Mjp$ with *Fⱼ* = 4, 7, 2, 5, 8, 3, 6, 1 can be achieved, taking advantage of the periodicity of the grating structure to effect a modulo-*p* measurement collection with phase stepping width enlarged by *M*. The value of *M* may be chosen in dependence on an expected size of the grating defects as will be explored in more detail. A particular advantage of this sequence is that if , for example, any five subsequent steps are corrupted by a defect in G, the remaining three will still cover reasonably well the range of full grating period of *p,* or, in angular terms, 2*π*. In the example sequence *Fⱼ* above, a good spread over the full period is achieved at increments of about 135°, which is a good approximation of equidistant sampling at 120°.

In order to achieve even better uniform sampling, the golden ratio $φ = \left(\sqrt{5}+1\right) / 2$ may be usefully employed herein. Specifically, the phase stepping width in (3) may be enlarged by (*φ* +*M*)*p* . This may yield a near uniform sampling of the phase 2π ([*j*((*φ* +*M*)*p*] mod *p*), and *j* = 1,2... Optionally, the number of phase steps *j* =0,2,..*N*-1, may be configured as *N* a Fibonacci number to achieve an even better approximation of equidistant sampling with enlarged step width caused by term (*φ* +*M*)*p*.

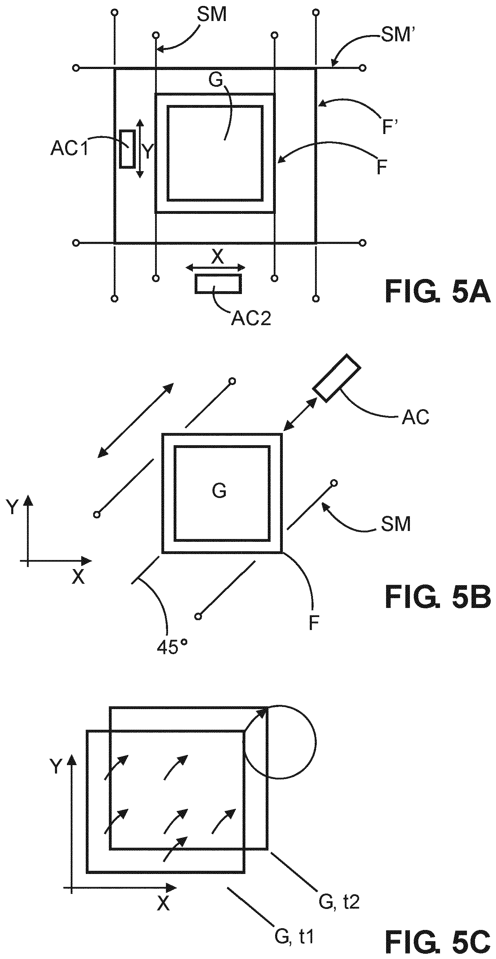

With reference to Fig. 5, there are shown, in projection views along direction Z, schematic block diagrams of various embodiments of suitable phase scanning mechanisms PSM configured to implement the above described phase stepping strategies. The scan paths as implemented by the phase scanning mechanism PSM is either lineal or curved.

Turning first to the embodiments for implementing a lineal scan path, reference is made to Fig. 5A which shows a phase scanning mechanism according to one embodiment. The grating G to be scanned is suitably arranged in inner frame F, and the inner frame F is mounted in an outer frame F'. Each of the frames F,F' are coupled to a respective slide mechanism SM,SM' that together allow independent motion into both X and Y direction. For example, a rail or slot mechanism may be used, with sliding facilitated by low friction coating. In addition or instead, the sliding motion of the frames, and with it the grating G, is mechanically facilitated by ball-bearing mechanisms such as rollers, etc. Respective actuators AC1, AC2 responsible for the respective motion in X and Y direction are arranged. The motor axes of the actuators AC1, AC2 are arranged to that the actuating force is exerted parallel to the X- and Y-direction, respectively. The actuators AC1, AC2 in the Fig. 5A-embodiment may operate concurrently, thus causing, by superposition, an effective lineal motion at an angle to both directions X, Y. A lineal motion may thus be effected at an angle to both directions X, Y to implement for instance a diagonal scan path of grating G. The displacement in Y direction facilitates phase stepping whilst the displacement in X direction facilities obviating pixel occlusion as explained above. Alternatively, but less preferred herein, the actuators AC1, AC2 may be activated in sequence with repeated phase stepping motions in Y direction interrupted by motions in X direction to obviate pixel occlusion by defective regions DR. In embodiments, if defects DR with size ${d}_{x}^{X}$ in X direction are negligible, the phase stepping motion along Y without displacement along X may be sufficient. A single actuator AC1 may then be used in the phase stepping mechanism PSM.

Fig. 5B shows a different embodiment where a single actuator AC may be used. In this embodiment, the scan path caused by the actuator is lineal and at 45° with respect to X and Y direction with the motor axis of the single actuator AC1 arranged at the said angle 45°. As in Fig. 5A, the grating G may be arranged in a frame F that has its corner portions slideably supported in the sliding mechanism SM. As above in Fig. 5A, a slot mechanism may be used instead. As in Fig. 5A, low friction coating or rollers or other mechanical facilitations may be used to promote smooth motion.

Fig. 5C shows a further embodiment, preferred herein, where the scan path is curved so that each point on the grid G proceeds on a curve. Preferably, the curve is a circular arc and the scan path is at 45° on said circular arc, with the center of rotation point located outside the grid G so as to preserve the orientation of the grid G parallel along X and Y directions when the grid G sweeps out the circular scan path. That is, the edges e1-e4 of the grating G remain parallel to the X and Y direction, whilst each point on the grating sweeps out its own circular arc. Fig. 5C shows the grating G at different time instants t1, t2 on the arcuate scan path.

In embodiments of Fig. 5, if the scan path is not parallel to the Y direction but is at an angle, it is preferable that for some or each step, the displacement component along direction Y is greater than p. The phase step width along component Y may be written as above as *p*(1/*N*+*M*) which is greater than the period *p,* but is not a multiple of the said period p. The displacement component along X is preferably large enough to avoid occlusion.

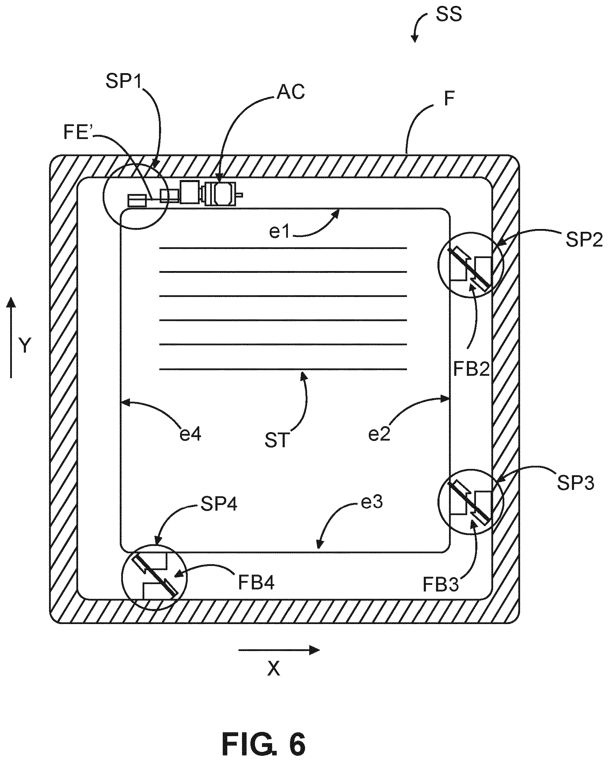

Fig. 6 is a more detailed illustration of an embodiment of Fig. 5C according to one embodiment. The grating G is suspended in a frame F at several suspension points SP1 to SP4, such as four suspension points SP1-SP4 as shown schematically as small circles in Fig. 6. The view afforded by Fig. 6 is along projection direction Z.

Advantageously, the curved scan path can be implemented in this embodiment with a single actuator AC as will be explained now in more detail below. One of the suspension points, for example suspension point SP1, is at edge e1 at the said single actuator AC, whilst the other suspension points SP2-4 are arranged and distributed at some or all the other sides e2-e4 of the grid G. In the embodiment shown, the suspension points SP2-4 are arranged at neighboring edges e2, e3 of the grid but this may not necessarily be so in all embodiments where the suspension points are at opposing edges. Whilst three additional suspension points SP2-SP4 are shown, one at one edge e3 and two at the others at edge e2, this is not necessarily required and only two such suspension points at the same or at different edges e2-e4 of the grid, neighboring or opposed, may be sufficient in embodiments. Preferably, to promote better arcuate grating motion, the at least two suspension points are at neighboring sides, leaving one side e4 at large. Whilst merely two suspension points may in principle be sufficient, one SP1 being at the actuator AC, the other SP2 at one of the other edges e2-e4, opposite to or neighboring the edge e1 where the actuator is located, this is likely to lead to an unstable suspension and is less preferred herein. The motor axis of the actuator is movable in X-direction and is suitably coupled to edge e1 of frame F. The coupling is rigid such that an actuating force can be exerted on the frame when the actuator pulls along direction X. The pulling force is in the view of Fig. 6 to the right in positive X direction.

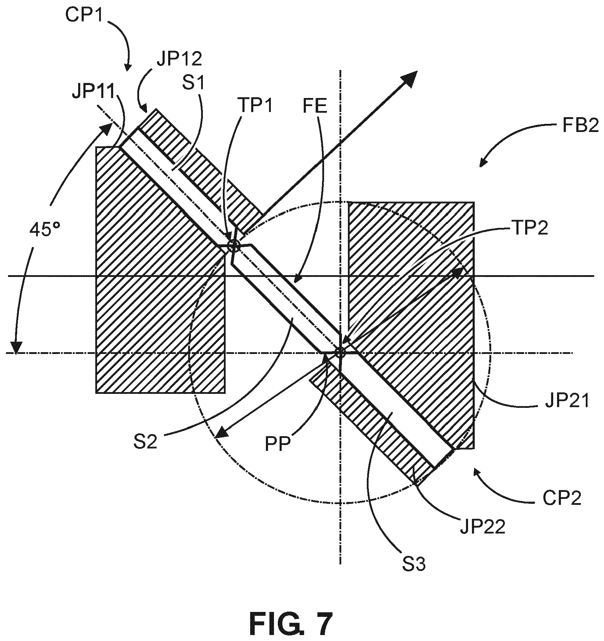

Preferably, suspension at suspension points SP2-SP4 other than at the actuator AC is implemented by respective flexure bearings FB2-FB4. One such flexure bearing FB2 is shown in more detail in Fig. 7. Fig. 7 affords a cross sectional view of the flexure bearing FB2 in a plane perpendicular to direction Z. The other flexure bearings FB3, FB4 have preferably a similar structure. The flexure bearing FB2 includes two clamps CP1, CP2 arranged at opposed relationship respectively at the grating G and at the frame F. Each clamp CP1, CP2 includes a pair of jaw portions JP11, JP12 and JP21, JP22, respectively. A first jaw portion JP11, JP21 of each pair is respectively coupled to the grating G and frame F. The coupling may be through gluing, bolting or otherwise. The grating G itself is preferably mounted on a carrier substrate (not shown) of sufficient stiffness such as glass, and the grating is coupled to the respective jaw portion via the said carrier substrate.

The flexure bearing FB2 further includes a flexure element FE such a blade of spring metal or of other suitably flexible material. The flexure element FE is structured into three segments SE1, SE2, SE3 by having two thinned regions TP1,2. The thinned regions TP1, TP2 may be arranged as shown in Fig. 7 by cut-outs, such as opposed grooves formed on both sides of the flexure element. The outer segments S1, S3 are clamped into the clamped portions CP1, CP2. The outer segments S1, S3 are securely held by the respective pair of jaws JP11, JP12, JP21, JP22. Each of the two outer segments S1, S3 is held in engagement with the respective pair of jaws JP11, JP12, JP21, JP22 by gluing or bolting or other suitable fixation. The center segment S2 bridges a gap to extend between the two clamp portions CP, CP2. Instead of a flat spring or blade as shown in Fig. 7, a pin flexure may be used for the flexure element FE. A similar flexure element FE' is used for couple the grating G to the actuator AC at suspension point SP1.

Preferably, and as is shown in Fig. 7, each of the respective flexure elements FE at suspension points SP2-SP4 is held at 45° in the X, Y coordinate system. In operation, that is, during the grating's phase stepping, the actuator AC as shown in Fig. 6 exerts a lateral force, for instance a pulling force, along direction X. This lateral pulling force is translated by the clamps CP1, CP2 into a local bending at thinned portions TP1 and TP2 of the flexure element FE, around an axis that extends perpendicularly into the plane of the section drawing Fig 7. This axis passes through one of the thinned portions TP2 of the flexure element, giving rise there to a pivot point PP around which the flexure element pivots. The above applies to each of the flexure bearings FB2-FB4, each having such as pivot point PP (not shown for bearings FB3,4). The action of the single actuator AC will thus cause the concurrent pivoting about the three pivot points PP at each bearing FB2-FB4. As a result, the entire grating G moves along the circular scan path as per Fig. 5C at 45° in an imaginary reference circle as shown in the X,Y co-ordinate system in Fig. 7, whilst respective edges e1-4 remain parallel along the X and Y direction. As a result of the pivoting around PP, there is also bending of the flexure element about the other thinned potion TP1, the flexure element being thus deformed into an elongated S-shape, with the center segment S2 being preferably stiff enough not to bend. It is also flexure element FE's at suspension SP1 at actuator AC that is subjected to bending upon actuator AC action.

The frame F is mounted in the imaging apparatus and remains stationary whilst the grating G sweeps out the arc as enabled by the three pivot points. For instance, if the grating G is the analyzer grating G2, the frame F is mounted at the detector DT, whilst frame is mounted at the source XS if G is the source grating G0. Inducing, as arrangement in Fig. 7 does, a circular motion at 45° will cause displacement components in X and Y direction at the same magnitude and thereby leading to a uniform phase stepping along Y and displacement along X, perpendicular to phase stepping direction Y, to so avoid occlusion by the pixels. To ensure a sufficient number of non-occluded measurements (preferably at least three) across a grating period *p* can be collected by preferably each pixel, the length of the flexure element FE (and hence the diameter of the imaginary circle) and the displacement along X by the actuator AC will need to be adjusted as described above. In particular, displacement value *M* and the sizes of the defects need to be taken into account, either through a priori knowledge or by using the defect evaluator DE. The angle of the arc swept out by the grating is in the order of 10°, but again this will depend on the size of the defects whose occluding effects one wishes to eliminate or at least reduce.

In sum, the three (or in embodiments two) flexure bearings FB2-4 arranged as shown allow pure translational motion (in particular, there is no rotation) of the frame with the grating G on the circular scan path. The flexure elements FE of the bearings FB2-4 are preferably held parallel to each other by their clamps and at the desired angle such as 45° in the respective X, Y coordinate system.

The arrangement in Figs. 5C, 6 and 7 is particularly advantageous if the imaging axis Z is horizontal as shown in Fig. 1 in which case gravity can be used to stabilize the motor axis of actuator AC so as to reduce unnecessary mechanical play.

In all of the above, including the embodiments with diagonal scan path in Figs. 5A,B, it is not necessary herein to strictly adhere to 45°, and allowance to either side of this target angulation is envisaged herein. In other words, a rage of about 35°-55° is preferably envisaged herein in embodiments of suitable angulations.

It should be noted that an arcuate scan path may also be implemented by the embodiment in Fig. 5A, by coordinating the incremental displacements in X and Y direction accordingly to cause a good stepped approximation of an arcuate path. However, in this embodiment, two actuators AC may be required, whilst in the embodiment of Figs. 5C,6, thanks to the new suspension mechanisms of Fig. 7, only a single such actuator AC is required. Costs can be reduced.

Reference is now made to the flow chart in Fig. 8 which shows steps S810-S860 of a process for phase contrast and/or dark field imaging.

At step S830 a phase stepping operation is performed by displacing a DAX/Φ-imaging facilitator device IFD along a scan path during an acquisition operation by an X-ray imaging apparatus to so acquire a sequence of intensity measurements per detector pixel. This phase stepping operation may also be referred to herein as an "object scan" as the object to be imaged is residing in the examination region during the phase stepping. The displacements are imparted on the imaging facilitator device IFD in discrete steps, referred to herein as phase steps. The imaging facilitator device IFD may include a grating having a suitable periodicity that depends on the mean energy of the X-radiation generated by an X-ray source of the X-ray imaging apparatus. The imaging facility structure is exposed to X-radiation during the phase-stepping. Alternatively, the phase stepping operation is imparted in a continuous displacement rather than in steps. Alternatively, the phase stepping in the object scan is done moving the focal spot or by collimator control to cause a sequence of openings.

At step S840, the sequence of measurements is received at a computing device including a processor.

At step S850, an image generation algorithm, such as a phase retrieval algorithm, is applied to the measurements by the computing device to obtain a phase contrast and/or dark field image. The algorithm includes fitting, for each pixel position, in a fitting operation, a signal model to some or all of the measurements per pixel. The signal model includes reference data. The reference data is dependent on the phase step. In other words, in the fitting operation there is a functional dependency of the reference data on the phase step.

At step S860, the dark field and/or phase image is then provided for display, or other processing or storage.

Preferably, the phase step width in the phase stepping operation of step S830 or the total distance by which the grating displaced, is greater than, or greater than a multiple of, the respective periodicity of the sub-structures of the imaging facilitator device IFD.

Preferably, the phase stepping operation at step S830 is along a curved path, e. g., a circular arc. Alternatively, the scan path is lineal. The path may be at an angle other than 90° relative to the course of longitudinal periodic sub-structure of the imaging facilitator device IFD. The substructures may include grating structures such as a series of longitudinal lamellae or trenches of a grating.

Preferably, in step S830, the scan path is such that it includes displacement components in both X and Y direction so as to enable, not only the phase stepping operation, but also simultaneously moving the grating in a manner so that detector pixel positions are not occluded at all times during the phase stepping procedure. Preferably it is thereby ensured that each pixel can record a sufficient number of different measurements (preferably at least three, better four or more non-occluded measurements), or at least that the number of such non-occluded pixels can be increased. Ensuring that a sufficient number of non-occluded measurements are so collected increases robustness of the image generation at step S850 and reduces image artifacts.

Optionally, the method may include an initial step S820 where a suitable step width for the phase stepping, and thus the distance covered during the phase stepping operation, is determined. In embodiments, this step includes acquiring a visibility map and evaluating the visibility map to establish an average or maximum size in X and/or Y direction for projection footprints of grating defects recorded in the visibility map. Based on this evaluation, the phase stepping width is set. The evaluation may be based on intensity value thresholding. Singular measurements below a given intensity are deemed to be part of a footprint of a grating defect. The phase stepping width may be set in a program a controller of an actuator to effect the step width and thus the total displacement of the imaging facilitator device for the desired number of step.

As a further option, the method includes a further phase stepping operation S810, performed before the phase stepping S830 during imaging in the object scan. This further phase stepping may be referred to herein as a "blank scan" as this is performed without the object to be imaged being present in the examination region of the imaging apparatus. For the measurements collected in the blank scan, a phase retrieval (see below at eq (5) ) is performed to obtain the reference data. The reference data is recorded in dependence on phase step *j*. For example, the intensity measurements per pixel may be indexed by the respective phase step *j*. The scan path is preferably the same as used in step S830 with step width adjusted to obviate occluded pixels. Alternatively, a standard phase stepping along Y direction (perpendicular to the longitudinal sub-structures) is used.

Optionally, pixel measurements affected by grating defect occlusion may be discarded. Alternatively, the so affected measurements are retained in the reference data. Optionally, the affected measurements are down-weighted relative to the non-occluded ones. Whether or not there is occlusion by grating defects can be established by intensity thresholding as in the step width determination step S820. Optionally, the visibility map as used in step S820 may be obtained from the reference data as acquired in the blank scan acquisition step S810.

Referring now in more detail to the image generation step S850, phase curves (measurement *Mⱼ* over the phase steps *j*) for each pixel/geometrical ray *i* can be respectively analyzed, for instance by fitting to a sinusoidal signal model as described in Pfeiffer et al Hard-X-ray dark-field imaging using a grating interferometer", published in Nature Materials 7, pp 134-137 (2008), to effect the image generation. Preferably, there are at least three fitting parameters *Tᵢ, Dᵢ, ϕᵢ* included in the three-channel sinusoidal model. The three fitting parameters represent, respectively, the three contributions phase-contrast, dark-field signal and attenuation. The sinusoidal model is fitted by image generator IGEN to the phase curves to so compute in particular the DAX- and/or Φ-image, and an attenuation (also called "transmission") image, although this is of lesser interest herein. Computation of the apparently superfluous transmission image may be required to correctly account for the three contrast effects as otherwise incorrect contributions are incurred in the DAX and/or Φ-channel.

An optimization procedure is used to fit the measured series of projections to the model. The procedure can be understood in terms of as cost function and the fitting operation can be formulated as an optimization problem. Any suitable optimization scheme such as gradient descent, conjugate gradients, Newton-Raphson, stochastic gradients, Maximum Likelihood approach, other statistical techniques or others are also envisaged. Non analytical methods such as neural networks or other Machine Learning techniques may also be used.

The optimization problem underlying the phase retrieval algorithm may be formulated in embodiments as: ${Δ}_{i} \left({T}_{i}, {D}_{i}, {ϕ}_{i}\right) = {\sum }_{j} {w}_{ij} {\left({M}_{ij}-{A}_{ij}{T}_{i}\left(1+{D}_{i}{V}_{ij}cos\left({ϕ}_{i}-{ψ}_{ij}\right)\right)\right)}^{2}$

Specifically, the task in the optimization is to improve the cost function Δ by adjusting the fitting parameters (*T, D, ϕ*). In this case, the parameters are to be adjusted in the optimization so that the values ("the cost") returned by the cost function Δ decreases. More than three channels may be used in signal model, depending on the number of contrast mechanism one wishes to account for.

Due to the phase stepping scheme as envisaged herein, the reference values of the reference data are now phase step-*j*-dependent. Thus, a "conventional" phase stepping S810 may be executed for each pixel *i,* resulting in the reference values *Aᵢⱼ, Vᵢⱼ,* and *ψᵢⱼ* or each image pixel *i* and phase step *j*. The phase retrieval S850 for the object scan (with object OB in the examination region) is then done by pixel-wise minimization of the cost function Δ*ᵢ*.

In yet more detail, the reference data includes reference values *Aᵢⱼ, Vᵢⱼ,* and *ψᵢⱼ*. Similar to (4), the reference values are also obtained by a similar signal model fitting procedure to the data collected in the blank scan. More specifically still, for each stepping position *j*, another series of *N'* measurements *Bᵢⱼₖ* without object in the examination region are collected. The reference values are obtained by minimizing for each pixel *i* and each desired distributed phase stepping location *j* the following cost: ${Δ}_{ij}^{′} \left({A}_{ij}, {V}_{ij}, {ψ}_{ij}\right) = {\sum }_{k} {w}_{ijk} {\left({B}_{ijk}-{A}_{ij}\left(1+{V}_{ij} cos\left({ψ}_{ij}-\frac{2 πk}{N ′}\right)\right)\right)}^{2}$

In eqs (4),(5), the *w*ᵢⱼₖ, *w*ᵢⱼ terms are optional weighting factors, such as correlation factors.

For the collection of the reference data in the blank scan, the above mentioned block stepping strategy may be used where the grating G starts at (x₀,y₀), then at (x₀',y₀'), etc.

When a distributed phase stepping strategy is used for the object scan, more reference data may need to be acquired in the blank-scan for each of the distributed stepping points, as opposed to the block-stepping strategy where the reference data needs to be collected only for the two (or more) starting positions (x₀,y₀), (x₀',y₀') as used in a block-stepping strategy.

As mentioned above, G0 may be stepped because it has the smallest size and is therefore easier to step. However, due to the large size and G2 being assembled from several grating M1-4 tiles of smaller size, G2 suffers more from defects than G0. Therefore, it is desired to combine the proposed approach with a stepping of G2.

It will be understood that for each grating G0-G2 that is to be stepped using the phase stepping strategies proposed herein, a respective set of reference data needs to be obtained with phase stepping in a blank scan and phase retrieval as per (5). Having a respective phase stepping mechanism as described above at Figs 5-7 for each of the grating G0-G2 may allow compensating for essentially all grating defects, but managing the resulting large amount of reference data may be undesirable for certain applications. In embodiments, it may therefore be preferably to phase step only the grating(s) that can be expected to include the most defects, such as the large grating G2 assembled from modules. Any single one, any two or, as said, all three gratings may be stepped with the proposed phase stepping strategies.

Instead of, or in addition to, the mechanical phase stepping mechanism PSM as described above in Figs. 5-7 for moving at least one of the gratings G, it is the focal spot FS that may be moved to effect the scan path relative to at least one of the gratings G. This can be achieved by using any one of the above mentioned embodiments, such as electrostatic deflection of the electron beam, etc. In embodiments with movable focal spot, all of the above described applies, with projective magnification factored in so that the measurements can be collected at the detector DT with the correct, that is, effective phase step width of greater than the period *p* or greater than a multiple of *p.* Preferably, phase stepping via focal spot FS movement may be done when collecting in step S810 the reference data, in particular for the source grating G0.

The components of the image processing system IPS, in particular the image generation algorithm IGEN and the actuator controller CL may be implemented as one or more software modules, run on one or more general-purpose processing units PU such as a workstation associated with the imager XI, or on a server computer associated with a group of imagers.

Alternatively, some or all components of the image processing system IPS or controller CL may be arranged in hardware such as a suitably programmed microcontroller or microprocessor, such an FPGA (field-programmable-gate-array) or as a hardwired IC chip, an application specific integrated circuitry (ASIC), integrated into the imager IS. In a further embodiment still, the image processing system may be implemented in both, partly in software and partly in hardware.

The different components of the image processing system may be implemented on a single data processing unit PU. Alternatively, some or more components are implemented on different processing units PU, possibly remotely arranged in a distributed architecture and connectable in a suitable communication network such as in a cloud setting or client-server setup, etc.

One or more features described herein can be configured or implemented as or with circuitry encoded within a computer-readable medium, and/or combinations thereof. Circuitry may include discrete and/or integrated circuitry, a system-on-a-chip (SOC), and combinations thereof, a machine, a computer system, a processor and memory, a computer program.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above-described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium (in particular, but not necessarily, a non-transitory medium), such as an optical storage medium or a solid-state medium supplied together with or as part of other hardware, but may also be distributed in other forms, such as via the internet or other wired or wireless telecommunication systems. Transitory storage media are also envisaged in embodiments.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and limiting. The invention is not limited to the disclosed embodiments. Other variations of the disclosed embodiments can be understood and effected by those skilled in the art in practicing the claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims, be they numerals, alphanumerical, or a combination of one or more letters, or a combination of any of the foregoing, should not be construed as limiting the scope.

## Claims

1. An imaging apparatus (IA) for dark-field X-ray imaging and/or phase-contrast-imaging, wherein the imaging apparatus is an apparatus for 2D imaging, such as a radiography apparatus, or an apparatus for 3D imaging such as a CT scanner apparatus,
the imaging apparatus including at least one device (G,IFD) for phase contrast and/or dark field imaging having a periodic structure with a spatial period, wherein the spatial period is along a first direction,
the imaging apparatus further including a phase stepping mechanism (PSM) configured, during X-ray exposure, to facilitate a relative phase stepping motion between the at least one device (G,IFD) and
a focal spot (FS) of an X-ray source (XS) of the imaging apparatus (IA), the relative phase stepping motion to cover a distance greater than the said spatial period,
wherein the displacement along the first direction has a step width greater than the spatial period.

2. Apparatus of claim 1, wherein the said distance is not a multiple of the said period or wherein the said distance is at least twice a pixel size of a detector of the imaging apparatus or wherein said distance is based on a size of a defective region (DR) in the periodic structure.

3. Apparatus of any one of the previous claims, wherein the phase stepping mechanism (PSM) is configured to impart the phase stepping motion in one or more steps having at least one width, wherein said at least one width is greater than, the said period.

4. Apparatus of any one of the previous claims, wherein said step width is based on a size of a defective region (DR) in the periodic structure.

5. Apparatus of claim 4, wherein the device is assembled from sub-modules (M1-4) giving rise to one or more gaps in the device (G, IFD), and wherein the said defective region (DR) includes the said one or more gaps.

6. Apparatus of any one of the previous claims, wherein the phase stepping mechanism (PSM) includes a frame (F) in which the device (G, IFD) is suspended, and an actuator (AC) configured to cause the said phase stepping motion, preferably wherein there is a single such actuator (AC).

7. Apparatus of any one of the previous claims, wherein the spatial period (*p*) is along a first direction, wherein the phase stepping motion has a displacement component along a second direction at an angle to the first direction and a displacement component along said first direction.

8. Apparatus of any one of claims 6-7, wherein the phase stepping motion is along a curve.

9. Apparatus of any one of claims 6-8, wherein the device is suspended in the frame at at least two suspension points (SP2, SP3) by a respective flexure bearing (FB2, FB3), preferably wherein at least one of the flexure bearings (FB2,3) has a flexure element (FE) angled at 40-50° relative to the said first direction.

10. Apparatus of any one of the previous claims, wherein the device (G, IFD) is an interferometric grating.

## Patentansprüche

1. Bildgebungseinrichtung (IA) für Dunkelfeld-Röntgenbildgebung und/oder Phasenkontrastbildgebung, wobei die Bildgebungseinrichtung eine Einrichtung zur 2D-Bildgebung, wie beispielsweise eine Röntgeneinrichtung, oder eine Einrichtung zur 3D-Bildgebung, wie beispielsweise eine CT-Scaneinrichtung, ist,
wobei die Bildgebungseinrichtung mindestens eine Vorrichtung (G, IFD) für Phasenkontrast- und/oder Dunkelfeldbildgebung einschließt, die eine periodische Struktur mit einer räumlichen Periode aufweist, wobei die räumliche Periode entlang einer ersten Richtung verläuft,
wobei die Bildgebungseinrichtung ferner einen Phasenschrittmechanismus (PSM) einschließt, der dafür konfiguriert ist, während einer Röntgenbelichtung eine relative Phasenschrittbewegung zwischen der mindestens einen Vorrichtung (G, IFD) und
einem Brennfleck (FS) einer Röntgenstrahlenquelle (XS) der Bildgebungseinrichtung (IA) zu erleichtern, damit die relative Phasenschrittbewegung eine Strecke abdeckt, die größer ist als die räumliche Periode,
wobei die Verschiebung entlang der ersten Richtung eine Schrittweite aufweist, die größer ist als die räumliche Periode.

2. Einrichtung nach Anspruch 1, wobei die Strecke kein Vielfaches der Periode ist oder wobei die Strecke mindestens das Doppelte einer Pixelgröße eines Detektors der Abbildungseinrichtung beträgt oder wobei die Strecke auf einer Größe eines defekten Bereichs (DR) in der periodischen Struktur beruht.

3. Einrichtung nach einem der vorstehenden Ansprüche, wobei der Phasenschrittmechanismus (PSM) dafür konfiguriert ist, die Phasenschrittbewegung in einem oder mehreren Schritten zu verleihen, die mindestens einer Breite aufweisen, wobei die mindestens eine Breite größer ist als die Periode.

4. Einrichtung nach einem der vorstehenden Ansprüche, wobei die Schrittweite auf einer Größe eines defekten Bereichs (DR) in der periodischen Struktur beruht.

5. Einrichtung nach Anspruch 4, wobei die Vorrichtung aus Untermodulen (M1-4) zusammengesetzt ist, was einen oder mehrere Spalte in der Vorrichtung (G, IFD) hervorruft, und wobei der defekte Bereich (DR) den einen oder die mehreren Spalte einschließt.

6. Einrichtung nach einem der vorstehenden Ansprüche, wobei der Phasenschrittmechanismus (PSM) einen Rahmen (F), in dem die Vorrichtung (G, IFD) aufgehängt ist, und einen Stellantrieb (AC), der dafür konfiguriert ist, die Phasenschrittbewegung zu bewirken, einschließt, vorzugsweise, wobei es einen einzigen solchen Stellantrieb (AC) gibt.

7. Einrichtung nach einem der vorstehenden Ansprüche, wobei die räumliche Periode (*p*) entlang einer ersten Richtung verläuft, wobei die Phasenschrittbewegung eine Verschiebungskomponente entlang einer zweiten Richtung in einem Winkel zur ersten Richtung und eine Verschiebungskomponente entlang der ersten Richtung aufweist.

8. Einrichtung nach einem der Ansprüche 6 bis 7, wobei die Phasenschrittbewegung entlang einer Kurve erfolgt.

9. Einrichtung nach einem der Ansprüche 6-8, wobei die Vorrichtung im Rahmen an mindestens zwei Aufhängepunkten (SP2, SP3) durch ein jeweiliges Festkörpergelenk (FB2, FB3) aufgehängt ist, vorzugsweise, wobei mindestens eines der Festkörpergelenke (FB2, 3) ein Biegeelement (FE) aufweist, das um 40-50° im Verhältnis zur ersten Richtung abgewinkelt ist.

10. Einrichtung nach einem der vorstehenden Ansprüche, wobei die Vorrichtung (G, IFD) ein interferometrisches Gitter ist.

## Revendications

1. Appareil d'imagerie (AI) pour l'imagerie radiographique en champ sombre et/ou l'imagerie par contraste de phase, dans lequel l'appareil d'imagerie est un appareil d'imagerie 2D, tel qu'un appareil de radiographie, ou un appareil d'imagerie 3D tel qu'un scanner CT,
l'appareil d'imagerie incluant au moins un dispositif (G, IFD) pour l'imagerie par contraste de phase et/ou en champ sombre présentant une structure périodique avec une période spatiale, dans lequel la période spatiale est orientée le long d'une première direction,
l'appareil d'imagerie incluant en outre un mécanisme de déphasage (PSM) configuré, pendant l'exposition aux rayons X, pour faciliter un mouvement de déphasage relatif entre au moins un dispositif (G, IFD) et
un point focal (FS) d'une source de rayons X (XS) de l'appareil d'imagerie (IA), le mouvement de déphasage relatif pour couvrir une distance supérieure à ladite période spatiale,
dans lequel le déplacement de la matrice le long de la première direction présente une largeur de pas supérieure à la période spatiale.

2. Appareil selon la revendication 1, dans lequel ladite distance n'est pas un multiple de ladite période ou dans lequel ladite distance est au moins deux fois la taille d'un pixel d'un détecteur de l'appareil d'imagerie ou dans lequel ladite distance est basée sur la taille d'une région défectueuse (DR) dans la structure périodique.

3. Appareil selon l'une quelconque des revendications précédentes, dans lequel le mécanisme de déphasage (PSM) est configuré pour imprimer le mouvement de déphasage en une ou plusieurs étapes présentant au moins une largeur, ladite au moins une largeur étant supérieure à ladite période.

4. Appareil selon l'une quelconque des revendications précédentes, dans lequel ladite largeur d'étape est basée sur la taille d'une région défectueuse (DR) dans la structure périodique.

5. Appareil selon la revendication 4, dans lequel le dispositif est assemblé à partir de sous-modules (M1-4) donnant lieu à un ou plusieurs espaces dans le dispositif (G, IFD), et dans lequel ladite région défectueuse (DR) inclut lesdits un ou plusieurs espaces.

6. Appareil selon l'une quelconque des revendications précédentes, dans lequel le mécanisme de déphasage (PSM) inclut un cadre (F) dans lequel le dispositif (G, IFD) est suspendu, et un actionneur (AC) configuré pour provoquer ledit mouvement de déphasage, de préférence dans lequel il existe un seul actionneur de ce type (AC).

7. Appareil selon l'une quelconque des revendications précédentes, dans lequel la période spatiale (*p*) est le long d'une première direction, dans lequel le mouvement de déphasage comporte une composante de déplacement le long d'une deuxième direction selon un angle par rapport à la première direction et une composante de déplacement le long de ladite première direction.

8. Appareil selon l'une quelconque des revendications 6-7, dans lequel le mouvement de déphasage s'effectue le long d'une courbe.

9. Appareil selon l'une quelconque des revendications 6-8, dans lequel le dispositif est suspendu dans le cadre au niveau d'au moins deux points de suspension (SP2, SP3) par un palier flexible respectif (FB2, FB3), de préférence dans lequel au moins un des paliers flexibles (FB2, 3) présente un élément flexible (FE) incliné à 40-50° par rapport à ladite première direction.

10. Appareil selon l'une quelconque des revendications précédentes, dans lequel le dispositif (G, IFD) est un réseau interférométrique.
